# EUROPEAN PATENT SPECIFICATION

(11) **EP 2 409 701 B1**
(45) Date of publication and mention of the grant of the patent: **30.04.2014**
(21) Application number: 11174384.5
(22) Date of filing: 18.07.2011
(51) Int. Cl.: A61K 31/4365, A61K 9/50, A61K 9/20, A61P 7/02, A61P 9/00

(54) **Prasugrel granules with improved stability**
Prasugrel-Körner mit erhöhter Stabilität
Granules de prasugrel dotées d'une meilleure stabilité

(30) Priority: 19.07.2010 TR 201005900
(43) Date of publication of application: 25.01.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Öner, Levent, 34398 Istanbul (TR)
(74) Representative: Sevinç, Erkan

(56) References cited:
- EP-A1- 2 100 607
- EP-A1- 2 275 087
- WO-A1-2010/094471
- US-A1- 2009 291 138

## Description

### Field of Invention

The present invention relates to improving the stability of prasugrel or a pharmaceutically acceptable salt of prasugrel. The present invention more particularly relates to a coating agent and a coating process, making prasugrel hydrochloride-containing granules more stable against air- and humidity-like ambient influences.

### Background of Invention

Prasugrel hydrochloride is a member of the thienopyridine class and inhibits the activation and aggregation of platelets by means of P2Y12 and ADP receptors. Its chemical designation is 2-acetoxy-5-(a-cyclopropylcarbonyl-2-fluorobenzyl)-4,5,6,7-tetrahydrothieno[3,2-c]pyridine hydrochloride, with the chemical structure illustrated below with Formula 1.

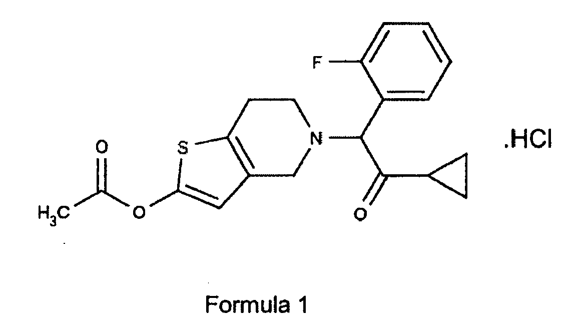

Prasugrel hydrochloride is a white solid. Prasugrel hydrochloride is well soluble at pH 2, weakly soluble at pH 3 to 4, and is substantially insoluble at pH 6 to 7.5.

Prasugrel hydrochloride is marketed under the trademark Effient^{®} in 5 or 10 mg unit dosage forms. It is initially administered in a dosage amount of 60 mg, as a loading dose. It is used in preventing or treating thrombosis and cardiovascular diseases.

Searching the patent literature reveals various patents in relation to prasugrel.

Patent application EP1298132, for instance, discloses a prasugrel hydrochloride salt, a method for obtaining this salt, and its use for thrombosis and embolism.

Patent application EP1728794 discloses a prasugrel maleate salt, a method for obtaining this salt, and its use for thrombosis and embolism.

Patent application WO2006135605 discloses a formulation containing prasugrel hydrochloride, packaged in an air and moisture impervious gas-inerted blister pack.

Patent application WO2006135605 discloses a dosage administration required for the loading dose and following doses of prasugrel.

Patent application WO2008140459 discloses a tablet or capsule formulation containing prasugrel, packaged in an air- and humidity-impermeable blister under a positive liquid gas pressure in order to reduce the amount of oxygen and humidity generated during packaging.

US 2009/291138-A1 discloses film coated preparations comprising prasugrel or prasugrel HCl and a film layer which is coated on the formulation, wherein film coating agents are selected from polyvinyl alcohol, sodium carboxymethyl cellulose and pullulan. According to the exemplary embodiments and stability tests, a stability value of max. 94% is achieved within 3 weeks.

EP 2 100 607-A1, discloses various formulations coated with conventional agents including hydroxypropylmethyl cellulose, lactose, titanium oxide, triacetin and water. According to the previous document US 2009/291138-A1, these conventional coating agents give a stability performance of around 78%.

EP 2 275 087-A1 describes Prasugrel controlled release formulations comprising prasugrel particles and one or more waxy substances or compounds such that prasugrel particles are embedded in a hydrophilic or hydrophobic matrix governing the release profile. As a side feature, this document provides encapsulation or coating of the prasugrel hydrochloride salt particles with cellulose or cellulose derivatives such as hydroxypropylmethylcellulose, hydroxypropylcellulose, or polyvinylpyrrolidone, which are reported to be as stability enhancers.

WO 2010/094471-A1 discloses a pharmaceutical composition comprising prasugrel, prepared in the absence of water and characterized by not containing lactose or mannitol. More specifically, teaching of this document seeks for the solution of stability problems by way of proposing a process which does not involve lactose and mannitol. This document does not propose use of combination of coating agents including pullulan and the specific methacrylate copolymer of the present invention. Although, methacrylate copolymers are generically addressed as suitable coating agents for the formulation, no clear specification or hint is given with respect to particular methacrylate copolymers, or combination thereof with pullulan.

Stability-related problems do occur in a plurality of active agents, including prasugrel, under the influence of ambient and physical conditions. Prasugrel is an active agent that is highly-susceptible to air and humidity. When prasugrel is exposed to air and humidity, it degrades structurally and develops behavioral changes. As a result of this fact, two main problems emerge. The first problem is that the stability of prasugrel products developed is not of desired level and the shelf life thereof is shortened. The seconds problem is that prasugrel is reactive against the excipients employed in developing formulations containing prasugrel. This fact causes impurities to occur in formulation and leads to incorporation of undesired components into the formulation.

Due to the abovementioned drawbacks, a novelty is required in the art of prasugrel hydrochloride formulations used for preventing and treating thrombosis and cardiovascular diseases.

### Object and Brief Description of Invention

The present invention relates to easily-administrable pregabalin granules, eliminating all aforesaid problems and brining additional advantages to the relevant prior art.

Accordingly, the main object of the present invention is to obtain a stable and coated granule with antithrombotic activity.

Another object of the present invention is to ensure the stabilization of prasugrel granules without the dissolution rate and bioavailability of the same being effected.

A further object of the present invention is to avoid aggregation and provide a desired level of flowability during production, thanks to granules obtained by coating the prasugrel hydrochloride granules with a convenient excipient.

Pharmaceutical granules containing prasugrel hydrochloride are obtained to carry out all objects, referred to above and to emerge from the following detailed description.

The novelty according to a preferred embodiment of the present invention is characterized in that said granules comprise at least one coating containing pullulan.

In a preferred embodiment according to the present invention, the amount of pullulan in said coating layer with respect to the total granule amount is 0.01 to 20% by weight, preferably 0.1 to 10% by weight, and more preferably 0.2 to 4% by weight.

In a preferred embodiment, there are preferably two coating layers provided.

In another preferred embodiment according to the present invention, said coating layer further comprises at least one or a mixture of hydroxypropyl methyl cellulose, hydroxypropyl cellulose, cellulose acetate phthalate, sodium carboxymethyl cellulose, methyl cellulose, hydroxypropyl methyl cellulose phthalate, hydroxypropyl methyl cellulose acetate succinate, polyvinyl acetate phthalate, methacrylic acid copolymers and polyvinyl alcohol.

In another preferred embodiment according to the present invention, said coating layer is preferably poly(butyl methacrylate-co-(2-dimethylaminoethyl) methacrylate-co-methyl methacrylate) 1:2:1.

A further preferred embodiment according to the present invention provides a method for preparing pharmaceutical granules, comprising the steps of
a. dissolving pullulan **and poly(butyl methacrylate-co-(2-dimethylaminoethyl) methacrylate-co-methyl methacrylate) 1:2:1** in a proper solvent,
b. spray-coating the solution obtained above onto the granules comprising prasugrel or a pharmaceutically acceptable salt of prasugrel, and
c. drying the coated granules.

### Detailed Description of Invention

A coating process has been realized that efficiently prevents the contact of granules with air and humidity in order to provide stability for the granules of prasugrel or a pharmaceutically acceptable salt of prasugrel. The process steps are as follows. Pullulan or an additional coating material (such as poly(butyl methacrylate-co-(2-dimethylaminoethyl) methacrylate-co-methyl methacrylate) 1:2:1) is dissolved in a proper solvent. The solution obtained is spray-coated onto the granules comprising prasugrel or a pharmaceutically acceptable salt of prasugrel, and the coating is realized. Then the coated granules are dried.

### Example

### Sachet formulation

| **Ingredients** | **weight (mg)** |
|---|---|
| prasugrel | 10 |
| microcrystalline cellulose | 35 |
| lactose monohydrate | 150 |
| mannitol | 225 |
| xylitol | 310 |
| flavoring agent | 5 |
| sucralose | 10 |
| colloidal silicone dioxide | 5 |
| microcrystalline cellulose-quar gum | 75 |
| microcrystalline cellulose - carboxymethyl cellulose sodium | 135 |
| **film coating layer** | |
| Pullulan | 15 |
| poly(butyl methacrylate-co-(2-dimethylaminoethyl) methacrylate-co-methyl methacrylate) 1:2:1. | 25 |

Coated prasugrel is mixed with microcrystalline cellulose, lactose monohydrate, mannitol, xylitol, flavoring agent, sucralose, colloidal silicone dioxide, microcrystalline cellulose-guar gum and microcrystalline cellulose - carboxymethyl cellulose sodium and this final mixture is then filled into alu - alu package.

### Stability Test Results

| **Samples** | **Remaining 4 weeks at 55 C° and % 85 relative humidity** |
|---|---|
| Amount of active ingredient of prasugrel | % 74 |
| Amount of active inredient with pullulan and poly(butyl methacrylate-co-(2-dimethylaminoethyl) methacrylate-co-methyl methacrylate) 1:2:1. (sachet formulation) | % 95 |

The coated granules surprisingly showed good dissolution rates and stability. This operation also avoids the aggregation of granules and any reduction in their flowability. The flowability of the granules obtained is high, and their humidity-permeability is low.

The term granule, as used herein, means a powder, particle, or pellet form of prasugrel or a pharmaceutically acceptable salt of prasugrel.

The present invention is used for preventing or treating thrombosis and cardiovascular diseases.

These granules can be used in the content of medicaments which are efficient in preventing or treating thrombosis and cardiovascular diseases.

The pharmaceutical compositions according to the present invention may also comprise one or more pharmaceutically acceptable excipients. Such pharmaceutically acceptable excipients include, but are not restricted to fillers, glidants, lubricants, disintegrants, surface active agents etc. and the mixtures thereof.

## Claims

1. Pharmaceutical granules comprising prasugrel hydrochloride, **characterized in that** said granules comprises at least one coating layer containing pullulan and poly(butyl methacrylate-co-(2-dimethylaminoethyl)methacrylate-co-methylmethacrylate) 1:2:1.

2. The pharmaceutical granules according to Claim 1, wherein the amount of pullulan in said coating layer with respect to the total granule amount is 0.01 to 20% by weight, preferably 0.1 to 10% by weight, and more preferably 0.2 to 4% by weight.

3. The pharmaceutical granules according to any of the preceding claims, wherein said coating is preferably made of two layers.

4. The pharmaceutical granules according to any of the preceding claims, wherein said granules are into the alu-alu package.

5. A method for preparing pharmaceutical granules, comprising the steps of
a. dissolving pulluian and poly(butyl methacrylate-co-(2-dimethylaminoethyl) methacrylate-co-methylmethacrylate) 1:2:1 in a proper solvent,
b. spray-coating the solution obtained above onto the granules comprising prasugrel or a pharmaceutically acceptable salt of prasugrel, and
c. drying the coated granules.

6. Use of pharmaceutical granules as claimed in any of the preceding claims in producing a medicament for the prevention or treatment of thrombosis and cardiovascular diseases.

7. Pharmaceutical granules according to any of the preceding claims for preventing or treating thrombosis and cardiovascular diseases in mammalians, but particularly in humans.

## Patentansprüche

1. Pharmazeutisches Granulat mit Prasugrelhydrochlorid,
**dadurch gekennzeichnet,**
**dass** das Granulat zumindest eine Hüllschicht aufweist, die Pullulan und Poly(butylmethacrylat-co-(2-dimethylaminoethyl)methacrylat-co-methylmethacrylat) im Verhältnis 1:2:1 enthält.

2. Pharmazeutisches Granulat nach Anspruch 1, wobei die Menge an Pullulan in der Hüllschicht in Bezug auf die Gesamtgranulatmenge 0,01 bis 20 Gew.-%, bevorzugt 0,1 bis 10 Gew.-%, und noch bevorzugter 0,2 bis 4 Gew.-% beträgt.

3. Pharmazeutisches Granulat nach einem der vorstehenden Ansprüche, wobei die Umhüllung bevorzugt aus zwei Schichten gefertigt ist.

4. Pharmazeutisches Granulat nach einem der vorstehenden Ansprüche, wobei das Granulat in der Alu-Alu-Verpackung ist.

5. Verfahren zum Herstellen von pharmazeutischem Granulat mit den Schritten:
a. Auflösen von Pullulan und Poly(butylmethacrylat-co-(2-dimethylaminoethyl) methacrylat-co-methylmethacrylat) im Verhältnis 1:2:1 in einem geeigneten Lösungsmittel,
b. Sprühbeschichten der oben erhaltenen Lösung auf das Granulat, das Prasugrel oder ein pharmazeutisch verträgliches Salz von Prasugrel enthält, und
c. Trocknen des beschichteten Granulates.

6. Verwendung von pharmazeutischem Granulat nach einem der vorstehenden Ansprüche bei der Herstellung eines Medikamentes für die Vermeidung oder Behandlung von Thrombosen und kardiovaskulären Krankheiten.

7. Pharmazeutisches Granulat nach einem der vorstehenden Ansprüche zur Vermeidung oder Behandlung von Thrombosen und kardiovaskulären Krankheiten bei Säugetieren, aber insbesondere bei Menschen.

## Revendications

1. Granulés pharmaceutiques comprenant du chlorhydrate de prasugrel, **caractérisés en ce que** lesdits granulés comprennent au moins une couche de revêtement contenant du pullulane et du poly(méthacrylate de butyl)-co-(méthacrylate de 2-diméthylaminoéthyl)-co-(méthacrylate de méthyl) 1 :2 :1.

2. Granulés pharmaceutiques selon la revendication 1, dans lesquels la quantité de pullulane dans ladite couche de revêtement par rapport à la quantité totale du granulé est de 0.01 à 20% en masse, de préférence de 0.1 à 10% en masse, et plus préférentiellement de 0.2 à 4% en masse.

3. Granulés pharmaceutiques selon l'une ou l'autre des revendications précédentes, dans lesquels ledit revêtement est constitué de préférence de deux couches.

4. Granulés pharmaceutiques selon l'une ou l'autre des revendications précédentes, dans lesquels lesdits granulés sont dans un emballage alu-alu.

5. Procédé de préparation de granulés pharmaceutiques comprenant les étapes consistant à
a) dissoudre le pullulane et le poly(méthacrylate de butyl)-co-(méthacrylate de 2-diméthylaminoéthyl)-co-(méthacrylate de méthyl) 1 :2 :1 dans un solvant approprié,
b) revêtir, par pulvérisation de la solution obtenue ci-dessus, les granulés comprenant du prasugrel ou un sel pharmaceutiquement acceptable du prasugrel, et
c) sécher les granulés revêtus.

6. Utilisation de granulés pharmaceutiques tels que revendiqués dans l'une ou l'autre des revendications précédentes pour la production d'un médicament pour la prévention ou le traitement de la thrombose et des maladies cardiovasculaires.

7. Granulés pharmaceutiques selon l'une ou l'autre des revendications précédentes pour la prévention ou le traitement de la thrombose et des maladies cardiovasculaires chez les mammifères, mais en particulier chez l'homme.
